# FASCICULE DE BREVET EUROPEEN

(11) **EP 0 737 508 B1**
(45) Date de publication et mention de la délivrance du brevet: **16.04.1997**
(21) Numéro de dépôt: 96400582.1
(22) Date de dépôt: 20.03.1996
(51) Int. Cl.: B01F 17/00, B01F 17/34, A61K 7/00, A61K 7/48

(54) **Utilisation d'un composé hydrofluorocarboné dans une émulsion, émulsion et composition comprenant un tel composé**
Verwendung einer Fluorkohlenwasserstoffverbindung in einer Emulsion, diese enthaltende Emulsion und Zusammensetzung
Use of hydrofluorocarbon compound in an emulsion, emulsion and composition containing the same

(30) Priorité: 11.04.1995 FR 9504336; 11.04.1995 FR 9504337
(43) Date de publication de la demande: 16.10.1996
(73) Titulaire: L'OREAL, 75008 Paris (FR)
(72) Inventeur: Philippe, Michel, 91320 Wissous (FR); Henrion, Jean-Christophe, 93500 Pantin (FR); Nicolas-Morgantini, Luc, 60810 Rully (FR); Dardenne, Agnès, 93600 Aulnay-sous-Bois (FR); Bollens, Eric, 94410 Saint-Maurice (FR)
(74) Mandataire: Miszputen, Laurent

(56) Documents cités:
- EP-A- 0 595 683
- EP-A- 0 609 131
- EP-A- 0 609 132
- EP-A- 0 629 394
- WO-A-93/11103
- GB-A- 1 598 567
- US-A- 3 993 744

## Description

La présente invention se rapporte à l'utilisation d'un composé hydrofluorocarboné comme agent stabilisant dans une émulsion ou comme composé essentiel de la phase grasse. L'invention concerne également une émulsion dont la phase grasse est essentiellement consitutée par le composé fluoré, ainsi qu'une composition consistant en ladite émulsion, notamment une composition cosmétique, hygiénique ou pharmaceutique.

Il est connu d'utiliser des perfluoropolyéthers notamment dans le domaine du nettoyage, de la protection et du maquillage de la peau ou des cheveux. Ces composés sont connus pour leur basse tension superficielle et leur facilité d'étalement, mais présentent une solubilité dans la plupart des fluides très réduite, excepté dans les fluides fluorés, ce qui rend très difficile leur formulation en cosmétique. Certains de ses composés, les perfluorométhylisopropyléthers, sont connus sous le nom de Fomblin HC, et sont commercialisés par MONTEFLUO.
Il est connu, par le document FR-A-2 684 668, des composés hydrofluorocarbonés présentant une bonne solubilité notamment dans les solvants classiques. Ils permettent l'obtention d'émulsions stables et homogènes. Notamment, la stabilité thermodynamique des émulsions comprenant ces composés, en particulier lorsqu'ils composent la totalité de la phase grasse, pourrait être améliorée.

On connaît également, par la demande EP-A-595 683, des émulsions eau-dans huile dont la phase huileuse comprend notamment un oléate de fluoroalkyle en C₇-C₁₀. Les émulsions comprennent un agent tensioactif siliconé afin d'améliorer leur stabilité. La demande GB-A-1598567 suggère des compositions pour le conditionnement des cheveux, pouvant comprendre, entre autres, un ester fluoré d'un acide organique et pouvant se présenter sous forme d'emulsion ou de lotion aqueuse. Des esters fluorés d'un acide organique sont aussi mentionnés, entre autres, dans le brevet US-A-3993744, comme composants actifs dans des compositions capillaires, à l'état dissous ou dispersé dans de l'eau. Selon GB-A-1598567 et US-A-3993744, ces esters fluorés peuvent être utilisés en combinaison avec des polysiloxanes.

La demanderesse s'est posée le problème d'améliorer encore la qualité et la stabilité d'émulsions, et ce quelque soit la nature des huiles employées.

La présente invention a pour but de proposer des composes hydrofluorocarbonés pour la préparation d'une émulsion de qualité et de stabilité améliorée par rapport à celles connues de l'état de la technique. Les composés hydrofluorocarbonés de l'invention peuvent en effet être incorporés comme agent stabilisant d'une émulsion comportant au moins une phase grasse et une phase aqueuse. Ils peuvent en outre être utilisés comme constituant essentiel de la phase grasse d'une émulsion.

L'invention a pour objet l'utilisation d'au moins un composé de formule (I) :

R_{F} - C₂H₄ - O - CO - R_{H} (I)

dans laquelle : R_{F} représente un groupement alkyle, linéaire ou ramifié, perfluoré ayant de 4 à 20 atomes de carbone, et
R_{H} représente un groupement alkyle, linéaire ou ramifié, ayant de 1 à 29 atomes de carbone,
en tant qu'agent stabilisant dans une émulsion comportant au moins une phase grasse et une phase aqueuse.

On a en effet constaté que la présence d'au moins un composé de formule (I), ou d'un mélange de ces composés, dans une émulsion, et notamment dans la phase grasse de l'émulsion, pouvait permettre d'améliorer la stabilité de ladite émulsion, quelque soit la nature chimique de la phase grasse employée.

Pour les composés de formule (I), le radical R_{F} peut de préférence représenter un groupement alkyle perfluoré ayant de 4 à 10 atomes de carbone et le radical R_{H} un groupement alkyle linéaire ou ramifié ayant de 1 à 15 atomes de carbone.
Parmi les composés de formule (I) utilisables dans l'émulsion selon l'invention, on peut citer l'hexanoate de 2-F-octyl éthyle, le 2-butyl-octanoate de 2'-F-hexyl éthyle, le 2-éthyl-hexanoate de 2'-F-hexyl éthyle, le 2-décyl-tétradécanoate de 2'-F-octyl éthyle, l'octanoate de 2-F-hexyl éthyle, l'octanoate de 2-F-octyl éthyle, l'octadécanoate de 2-F-octyl éthyle, le docosanoate de 2-F-octyl éthyle et le décanoate de 2-F-octyl éthyle.

Lorsque le composé de formule (I) est utilisé comme agent stabilisant, il peut être présent dans l'émulsion, de préférence dans la phase grasse, à raison de 0,1 à 30% en poids, de préférence 0,5-20% en poids.

La phase grasse de l'émulsion peut comprendre en outre au moins une huile, qui peut être choisie notamment parmi les huiles hydrocarbonées, éventuellement fluorées, les huiles siliconées et les huiles perfluorées, seules ou en mélange.

Le composé de formule (I) peut être présent, dans l'émulsion, en une quantité allant de 0,01 à 60% en poids et de préférence de 0,1 à 20% en poids par rapport au poids total de l'émulsion.

Les composés de formule (I) peuvent aussi être utilisés comme constituants essentiels de la phase grasse d'une émulsion.

On a en effet constaté que, de manière surprenante, le fait que la phase grasse comprenne essentiellement au moins un composé de formule (I), ou un mélange de composés de formule (I), pouvait permettre de stabiliser thermodynamiquement l'émulsion, dans le temps.

L'invention a aussi pour objet une émulsion comprenant au moins une phase grasse et une phase aqueuse, ladite phase grasse étant essentiellement constituée d'au moins un composé hydrofluorocarboné de formule (I).

La présente invention se rapporte également à une composition cosmétique, hygiénique ou pharmaceutique, voire alimentaire, consistant en une émulsion contenant au moins une phase aqueuse et une phase grasse essentiellement constituée d'au moins un composé de formule (I).

Par 'constitué essentiellement', on entend dans la présente description que la phase grasse comprend le composé de formule (I) en tant qu'huile liquide unique, ledit composé représentant de préférence au moins 50% en poids de ladite phase grasse; ladite phase grasse peut alors comprendre 0-50% d'additifs conventionnels lipophiles autres que les huiles. De préférence, le composé de formule (I), ou le mélange de ces composés représente au moins 75% en poids de la phase grasse.

Quelque soit l'utilisation du composé de formule (I), la phase grasse de l'émulsion peut comprendre des additifs liposolubles classiquement utilisés dans le domaine d'application, tels que les vitamines, les parfums, les acides gras, les lipides cireux et notamment les céramides.

La phase aqueuse des émulsions de l'invention peut comprendre également des additifs hydrophiles classiquement utilisés, tels que le glycérol et l'urée.

Le rapport phase aqueuse/phase huileuse dans les émulsions peut être compris entre 9 et 0,1, de préférence entre 5 et 1,5. L'émulsion peut ainsi se présenter sous forme eau-dans-huile ou huile-dans-eau, voire sous la forme d'une émulsion multiple.

Les émulsions selon l'invention peuvent être utilisées en tant que composition, notamment dans les domaines cosmétique, hygiénique et pharmaceutique, voire alimentaire. Ladite composition peut se présenter en particulier sous forme d'un gel, d'un lait ou d'une crème.

En particulier, elles peuvent se présenter sous la forme d'un produit destiné au maquillage et/ou au soin de la peau ou de matières kératiniques, ou encore sous la forme d'un produit de coloration du cheveu ou d'un produit de protection solaire de la peau et/ou des matières kératiniques.

Les compositions conformes à l'invention, consistant en des émulsions, dont la phase grasse est constituée essentiellement de composés de formule (I), peuvent comprendre tout additif autre qu'une huile usuellement utilisé dans le domaine considéré, tel que les tensioactifs, les agents hydratants, les solvants organiques, les silicones, les épaississants, les adoucissants, les filtres solaires, les agents de traitement, les agents anti-mousse, les parfums, les conservateurs, les agents antioxydants, les séquestrants, les agents de sapidité, les agents alcalinisants ou acidifiants, les charges et les pigments, des émulsionnants, des co-émulsionnants.

Le composé de formule (I) peut être préparé selon un procédé classique d'estérification qui consiste à mélanger, en milieu acide, de préférence en présence d'un solvant, un acide de formule R_{H} - COOH avec un alcool de formule R_{F} - C₂H₄ -OH, dans lesquels les radicaux R_{H} et R_{F} ont les mêmes significations que précédemment.

Un autre procédé d'estérification pour préparer le composé de formule(I) consiste à faire réagir un alcool de formule R_{F} - C₂H₄ - OH avec un dérivé activé d'acide de formule R_{H} - CO - Y dans lequel R_{H} a la même signification que précédemment, et Y représente un groupement activant, notamment de type halogène, résidu anhydride mixte ou symétrique, ou imidazole.

Les exemples qui suivent illustrent l'invention. Les exemples 1 à 7 décrivent un procédé de préparation de composés utilisés dans le cadre de la présente invention.

### EXEMPLES DE PREPARATION

### EXEMPLE 1 : Préparation du 2-éthyl-hexanoate de 2-F-hexyl éthyle

Dans un réacteur de 1 litre, on introduit 114,66 g de 2-F-hexyl éthanol et 43,2 g d'acide 2-éthyl-hexanoïque dans 850 ml de toluène en présence de 9 g d'acide paratoluène sulfonique monohydraté.
Le mélange réactionnel est porté au reflux du solvant pendant 16 heures. On concentre la solution pour obtenir une huile qui est distillée pour obtenir 113 g de composé ( rendement 77%).

L'analyse chimique du produit obtenu donne :
- Température d'ébullition : 115°C à 0,3 mbar
- Spectre RMN 13C : conforme à la structure attendue
- Spectre de masse : conforme à la structure attendue
- Analyse élémentaire :

| | % C | % H | % F |
|---|---|---|---|
| Théorique | 39,20 | 3,91 | 50,37 |
| Calculé | 39,23 | 3,81 | 50,42 |

### EXEMPLE 2 : Préparation de l'octanoate de 2-F-hexyl éthyle

Dans un réacteur de 2 litres, on dissout 43,2 g d'acide octanoïque et 114,7 g de 2-F-hexyl éthanol dans 850 ml de toluène en présence de 9 g d'acide paratoluène sulfonique. On chauffe le mélange au reflux pendant 16 heures, puis on évapore le solvant pour obtenir une huile qui est reprise par 800 ml d'éthanol. Cette solution est agitée pendant 15 minutes en présence de 150 g de résine préalablement lavée à l'eau, d'un mélange 50/50 éthanol/eau, puis d'éthanol seul. On filtre la résine sur verre fritté, puis on évapore le solvant pour obtenir une huile qui est distillée sous vide pour fournir 110 g du produit (rendement 75%).

L'analyse chimique du produit obtenu donne :
- Température d'ébullition : 105°C à 0,4 mbar
- Spectre de masse : conforme à la structure attendue
- Analyse élémentaire :

| | %C | %H | %F |
|---|---|---|---|
| Théorique | 39,20 | 3,91 | 50,37 |
| Calculé | 39,31 | 3,97 | 50,67 |

### EXEMPLE 3 : Préparation du 2-butyl-octanoate de 2-F-hexyl éthyle

Dans un réacteur de 1,5 litres, on introduit 76,44 g de 2-F-hexyl éthanol et 40 g d'acide 2-butyl-octanoïque dans 850 ml de toluène en présence de 6 g d'acide paratoluène sulfonique monohydraté.
Le mélange réactionnel est porté au reflux pendant 16 heures. On concentre la solution pour obtenir une huile qui est distillée pour obtenir le composé attendu (rendement 73%).

L'analyse chimique du produit obtenu donne :
- Température d'ébullition : 136 - 140°C à 0,6 mbar
- Spectre de masse : conforme à la structure attendue
- Spectre de RMN¹³C : conforme à la structure attendue
- Analyse élémentaire :

| | %C | %H | %F |
|---|---|---|---|
| Théorique | 43,96 | 4,98 | 45,20 |
| Calculé | 43,84 | 4,91 | 45,49 |

### EXEMPLE 4 : Préparation de 2-décyl-tétradécanoate de 2-F-octyl éthyle

Dans un réacteur de 1 litre, on introduit 69,6 g de 2-F-octyl éthanol et 46,8 g d'acide 2-décyl-tétradécanoïque dans 640 ml de toluène en présence de 4,49 g d'acide paratoluène sulfonique monohydraté.

Le mélange réactionnel est porté au reflux du solvant pendant 16 heures. On évapore le solvant pour obtenir une huile qui précipite par ajout de 500 ml d'éthanol. Le solide obtenu est filtré et lavé 2 fois à l'éthanol. Après séchage sous pression réduite, on obtient 78 g d'un solide blanc (rendement 76%).

L'analyse chimique du produit obtenu donne :
- Température de fusion : 43°C
- Spectre de masse : conforme à la structure attendue
- Spectre de RMN ¹³C : conforme à la structure attendue
- Analyse élémentaire :

| | %C | %H | %F |
|---|---|---|---|
| Théorique | 50,12 | 6,31 | 39,64 |
| Calculé | 50,06 | 6,26 | 39,64 |

### EXEMPLE 5 : Préparation de l'octadécanoate de 2-F-octyl éthyle

Dans un ballon de 1 litre, on dissout 69,6 g de 2-F-octyl éthanol dans 200 ml de tétrahydrofurane, puis on additionne 12,64 g de pyrridine. On refroidit la solution à 5°C et sous agitation on verse en 30 minutes 45,38 g de chlorure de stéaryle dans 100 ml de tétrahydrofurane. Puis on laisse revenir à température ambiante et on laisse agiter pendant 1 heure.
On concentre le mélange et on précipite le produit attendu par ajout de 500 ml d'éthanol. On filtre le solide obtenu qui est lavé et recristallisé dans l'éthanol. Après séchage sous pression réduite, on obtient 58 g d'un solide blanc ( rendement 53%).

L'analyse du produit obtenu donne:
- Température de fusion : 40°C
- Spectre de masse : conforme à la structure attendue
- Analyse élémentaire :

| | %C | %H | %F |
|---|---|---|---|
| Théorique | 46,03 | 5,38 | 44,21 |
| Calculé | 46,44 | 5,42 | 43,53 |

### EXEMPLE 6 : Préparation de l'hexanoate de 2-F-octyl éthyle

Dans un réacteur de 2 litres, on dissout 34,8 g d'acide hexanoïque et 146,16 g de 2-F-octyl éthanol dans 850 ml de toluène en présence de 9 g d'acide paratoluène sulfonique. On chauffe le mélange au reflux pendant 36 heures, puis on évapore le solvant pour obtenir une huile qui est reprise par 600 ml d'éthanol. Cette solution est agitée pendant 15 minutes en présence de 150 g de résine préalablement lavée avec de l'eau, d'un mélange 50/50 éthanol/ eau, puis d'éthanol seul. On filtre la résine sur verre fritté puis on évapore le solvant pour obtenir une huile qui est distillée sous pression réduite pour fournir 143 g (0,254 mole) de l'ester attendu (rendement 85%).

L'analyse chimique du produit obtenu donne :
- Température d'ébullition : 107 - 112°C à 0,4 mbar
- Spectre de masse : conforme à la structure attendue
- Spectre de RMN¹H et ¹³C : conforme à la structure attendue
- Analyse élémentaire

| | %C | %H | %f |
|---|---|---|---|
| Théorique | 34,18 | 2,69 | 57,44 |
| Calculé | 34,12 | 2,73 | 57,59 |

### EXEMPLE 7 : Préparation du docosanoate de 2-F-octyl éthyle

Dans un réacteur de 1 litre, on introduit 97,44 g de 2-F-octyl éthanol et 68 g d'acide docosanoïque dans 800 ml de toluène en présence de 6 g d'acide paratoluène sulfonique monohydraté.
Le mélange réactionnel est porté au reflux du solvant pendant 24 heures. On évapore le solvant pour obtenir une cire qui est recristallisée dans l'éthanol absolu. On obtient finalement 132,2 g d'un solide blanc (rendement 84%).

L'analyse chimique du produit obtenu donne :
- Température de fusion : 70,6°C
- Spectre de masse : conforme à la structure attendue
- Spectre de RMN¹³C : conforme à la structure attendue
- Analyse élémentaire :

| | %C | %H | %F |
|---|---|---|---|
| Théorique | 48,86 | 6,02 | 41,05 |
| Calculé | 49,02 | 6,01 | 40,81 |

### EXEMPLES COMPARATIFS ENTRE UNE EMULSION SELON L'INVENTION CONTENANT UN COMPOSE DE FORMULE (I) COMME AGENT STABILISANT ET UNE EMULSION DE L'ART ANTERIEUR

### EXEMPLE 8 : exemple comparatif

Cet exemple consiste à comparer la stabilité d'une émulsion stabilisée selon l'invention avec une émulsion stabilisée selon l'art antérieur (FR 2 684 668).

| *Art antérieur (% en poids):* | |
|---|---|
| . 1-(2'-F-hexyléthylthio) 3-(2''-éthylhexyloxy)-2-propanol | 4,5 % |
| . huile siliconée (cyclopentadiméthylsiloxane) | 45,5 % |
| . émulsionnant (polymère constitué de 80% d'acide méthacrylique et de 20% de méthacrylate de lauryle neutralisé à 80%) | 1 % |
| . eau | qsp 100 % |

| *Invention (% en poids) :* | |
|---|---|
| . composé de l'exemple 1 | 4,5 % |
| . huile siliconée (cyclopentadiméthylsiloxane) | 45,5 % |
| . émulsionnant (polymère 80% acide méthacrylique / 20% méthacrylate de lauryle, neutralisé à 80%) | 1 % |
| . eau | qsp 100 % |

La stabilité de l'émulsion selon l'invention est nettement supérieure comparée à celle de l'émulsion de l'état de la technique.
En effet, l'émulsion selon l'invention reste stable pendant plus de 100 jours, lors d'une conservation à 20°C, alors que l'émulsion selon l'état de la technique ne reste stable qu'environ 35 jours; on observe ensuite une démixtion de l'émulsion.

### EXEMPLE 9 : exemple comparatif

On compare la stabilité d'émulsions comprenant l'agent stabilisant selon l'invention, avec les mêmes émulsions sans stabilisant.

On obtient les résultats suivants :

### 1ère comparaison

| *Invention (% en poids)* | |
|---|---|
| . composé de l'exemple 1 | 10 % |
| . triglycérides d'acides caprylique et caprique | 40 % |
| . émulsionnant (polymère 80% acide méthacrylique / 20% méthacrylate de lauryle, neutralisé à 80%) | 1 % |
| . eau | qsp 100 % |

| *Art antérieur* | |
|---|---|
| . triglycérides d'acides caprylique et caprique | 50 % |
| . émulsionnant (polymère 80% acide méthacrylique / 20% méthacrylate de lauryle, neutralisé à 80%) | 1 % |
| . eau | qsp 100 % |

La stabilité de l'émulsion selon l'invention est nettement supérieure comparée à celle de l'émulsion de l'état de la technique. L'émulsion selon l'invention reste stable pendant plus de 80 jours, lors d'une conservation à 20°C, alors que l'émulsion selon l'état de la technique ne reste stable qu'environ 9 jours dans les mêmes conditions.

### 2ème comparaison

| *Invention (% en poids)* | |
|---|---|
| . composé de l'exemple 2 | 10 % |
| . ester palmitique de l'éthyl-2-hexyl glycéryl éther | 40 % |
| . émulsionnant (polymère 80% acide méthacrylique / 20% méthacrylate de lauryle, neutralisé à 80%) | 1 % |
| . eau | qsp 100 % |

| *Art antérieur* | |
|---|---|
| . ester palmitique de l'éthyl-2-hexyl glycéryl éther | 50 % |
| . émulsionnant (polymère 80% acide méthacrylique / 20% méthacrylate de lauryle, neutralisé à 80%) | 1 % |
| . eau | qsp 100 % |

La stabilité de l'émulsion selon l'invention est nettement supérieure comparée à celle de l'émulsion de l'état de la technique. L'émulsion selon l'invention reste stable pendant plus de 80 jours, lors d'une conservation à 20°C, alors que l'émulsion selon l'état de la technique ne reste stable qu'environ 45 jours dans les mêmes conditions.

### 3ème comparaison

| *Invention (% en poids)* | |
|---|---|
| . composé de l'exemple 1 | 10 % |
| . cyclopentadiméthylsiloxane | 40 % |
| . émulsionnant (polymère 80% acide méthacrylique / 20% méthacrylate de lauryle, neutralisé à 80%) | 1 % |
| . eau | qsp 100 % |

| *Art antérieur* | |
|---|---|
| . cyclopentadiméthylsiloxane | 50 % |
| . émulsionnant (polymère 80% acide méthacrylique / 20% méthacrylate de lauryle, neutralisé à 80%) | 1 % |
| . eau | qsp 100 % |

La stabilité de l'émulsion selon l'invention est nettement supérieure comparée à celle de l'émulsion de l'état de la technique. L'émulsion selon l'invention reste stable pendant plus de 15 jours, lors d'une conservation à 20°C, alors que l'émulsion selon l'état de la technique ne reste stable qu'environ 7 jours dans les mêmes conditions.

### EXEMPLES D'EMULSION DONT LA PHASE GRASSE EST ESSENTIELLEMENT CONSTITUEE D'UN COMPOSE DE FORMULE (I)

### Exemple A : exemple d'émulsion

On prépare une émulsion comprenant (% en poids):

| | |
|---|---|
| . Composé de l'exemple 1 | 50 % |
| . Emulsionnant (polymère constitué de 80% d'acide méthacrylique et de 20% de méthacrylate de lauryle neutralisé à 80%) | 1 % |
| . Eau | qsp 100 % |

L'émulsion obtenue se présente sous forme visqueuse, opaque, homogène et opalescente. La taille des globules est d'environ 0,5 µm.

### Exemple B : exemple d'émulsion

On prépare une émulsion comprenant (% en poids):

| | |
|---|---|
| . Composé de l'exemple 2 | 50 % |
| . Emulsionnant (polymère constitué de 80% d'acide méthacrylique et de 20% de méthacrylate de lauryle neutralisé à 80%) | 1 % |
| . Eau | qsp 100 % |

L'émulsion obtenue se présente sous forme visqueuse, opaque, homogène et opalescente. La taille des globules est d'environ 0,5 µm.

### EXEMPLE C : exemple comparatif

Cet exemple consiste à comparer la stabilité d'une émulsion selon l'invention (exemple A) avec une émulsion de l'art antérieur (FR 2 684 668) ayant la composition suivante (% en poids) :

| | |
|---|---|
| . 1-(2'-F-hexyléthylthio)3-(2''-éthylhexyloxy)-2-propanol | 50 % |
| . Emulsionnant (polymère constitué de 80% d'acide méthacrylique et de 20% de méthacrylate de lauryle neutralisé à 80%) | 1 % |
| . Eau | qsp 100 % |

La stabilité de l'émulsion selon l'invention est nettement supérieure comparée à celle de l'émulsion de l'état de la technique.
En effet, l'émulsion selon l'invention reste stable pendant plus de 20 jours, lors d'une conservation à 20 °C, alors que l'émulsion selon l'état de la technique ne reste stable qu'environ 15 jours; on observe ensuite une démixtion de l'émulsion.

## Revendications

1. Utilisation d'au moins un composé hydrofluorocarboné de formule (I) :
R_{F} - C₂H₄ - O - CO - R_{H}
dans laquelle : R_{F} représente un groupement alkyle, linéaire ou ramifié, perfluoré ayant de 4 à 20 atomes de carbone,
R_{H} représente un groupement alkyle, linéaire ou ramifié, ayant de 1 à 29 atomes de carbone,
comme agent stabilisant dans une émulsion comportant au moins une phase grasse et une phase aqueuse.

2. Utilisation selon la revendication 1, dans laquelle R_{F} représente un groupement alkyle perfluoré ayant 4 à 10 atomes de carbone.

3. Utilisation selon l'une des revendications précédentes, dans laquelle R_{H} représente un groupement alkyle linéaire ou ramifié ayant 1 à 15 atomes de carbone.

4. Utilisation selon l'une des revendications précédentes, dans laquelle le composé de formule (I) est choisi parmi l'hexanoate de 2-F-octyl éthyle, le 2-butyl-octanoate de 2'-F-hexyl éthyle, le 2-éthyl-hexanoate de 2'-F-hexyl éthyle, le 2-décyl-tétradéca-noate de 2'-F-octyl éthyle, l'octanoate de 2-F-hexyl éthyle, l'octanoate de 2-F-octyl éthyle, l'octadécanoate de 2-F-octyl éthyle, le docosanoate de 2-F-octyl éthyle et le décanoate de 2-F-octyl éthyle.

5. Utilisation selon l'une quelconque des revendications précédentes, dans laquelle l'agent stabilisant est présent dans la phase grasse de l'émulsion.

6. Utilisation selon l'une quelconque des revendications précédentes, dans laquelle le composé de formule (I) est présent dans l'émulsion en une quantité de 0,1 à 30% en poids.

7. Utilisation selon la revendication 6, dans laquelle le composé de formule (I) est présent dans l'émulsion en une quantité de 0,5-20% en poids.

8. Utilisation selon l'une quelconque des revendications précédentes, dans laquelle la phase grasse de l'émulsion comprend au moins une huile, qui peut être choisie parmi les huiles hydrocarbonées, éventuellement fluorées, les huiles siliconées, les huiles perfluorées, seules ou en mélange.

9. Utilisation selon l'une quelconque des revendications précédentes, dans laquelle l'émulsion est une émulsion huile-dans-eau ou eau-dans-huile ou une émulsion multiple.

10. Utilisation selon l'une quelconque des revendications précédentes, dans laquelle le rapport phase aqueuse/phase huileuse est compris entre 9 et 0,1, de préférence entre 5 et 1,5.

11. Utilisation selon l'une quelconque des revendications précédentes, dans un ou pour la préparation d'un produit cosmétique, hygiénique, pharmaceutique ou alimentaire.

12. Utilisation selon l'une quelconque des revendications précédentes, dans un ou pour la préparation d'un produit de maquillage et/ou de soin de la peau ou de matières kératiniques, ou un produit de coloration du cheveu ou un produit de protection solaire de la peau et/ou des matières kératiniques.

13. Emulsion comprenant une phase grasse et une phase aqueuse, caractérisée en ce que la phase grasse comprend, en tant qu'huile liquide unique, au moins 50 % en poids, par rapport au poids total de la phase grasse, d'au moins un composé hydrofluorocarboné de formule (I), tel que défini dans l'une des revendications 1 à 4.

14. Emulsion selon la revendication 13, dans laquelle le composé de formule (I), ou le mélange de ces composés, représente au moins 75% en poids de la phase grasse.

15. Emulsion selon l'une des revendications 13 ou 14, dans laquelle la phase grasse comprend en outre au moins un additif liposoluble de préférence choisi parmi les vitamines, les parfums, les acides gras, les lipides, les céramides.

16. Emulsion selon l'une des revendications 13 à 15, dans laquelle le rapport phase aqueuse/phase huileuse est compris entre 9 et 0,1, de préférence entre 5 et 1,5.

17. Emulsion selon l'une des revendications 13 à 16, se présentant sous forme eau-dans-huile ou huile-dans-eau, ou sous la forme d'une émulsion multiple.

18. Composition cosmétique, hygiénique, pharmaceutique ou alimentaire, caractérisée en ce qu'elle consiste en une émulsion selon l'une des revendications 13 à 17.

19. Composition selon la revendication 18 se présentant sous la forme d'un produit de maquillage et/ou de soin de la peau ou de matières kératiniques, ou sous la forme d'un produit de coloration du cheveu ou d'un produit de protection solaire de la peau et/ou des matières kératiniques.

## Claims

1. Use of at least one fluorohydrocarbon compound of formula (I):
R_{F}-C₂H₄-O-CO-R_{H} (I)
in which: R_{F} represents a linear or branched perfluorinated alkyl group having from 4 to 20 carbon atoms,
R_{H} represents a linear or branched alkyl group having from 1 to 29 carbon atoms,
as stabilizing agent in an emulsion containing at least one fatty phase and one aqueous phase.

2. Use according to Claim 1, in which R_{F} represents a perfluorinated alkyl group having 4 to 10 carbon atoms.

3. Use according to either of the preceding claims, in which R_{H} represents a linear or branched alkyl group having 1 to 15 carbon atoms.

4. Use according to one of the preceding claims, in which the compound of formula (I) is chosen from 2-(F-octyl) ethyl hexanoate, 2'-(F-hexyl)ethyl 2-butyloctanoate, 2'-(F-hexyl)ethyl 2-ethylhexanoate, 2'-(F-octyl)ethyl 2-decyltetradecanoate, 2-(F-hexyl)ethyl octanoate, 2-(F-octyl)ethyl octanoate, 2-(F-octyl)ethyl octadecanoate, 2-(F-octyl)ethyl docosanoate and 2-(F-octyl)ethyl decanoate.

5. Use according to any one of the preceding claims, in which the stabilizing agent is present in the fatty phase of the emulsion.

6. Use according to any one of the preceding claims, in which the compound of formula (I) is present in the emulsion in an amount of 0.1 to 30 % by weight.

7. Use according to Claim 6, in which the compound of formula (I) is present in the emulsion in an amount of 0.5-20 % by weight.

8. Use according to any one of the preceding claims, in which the fatty phase of the emulsion comprises at least one oil which can be chosen from hydrocarbon oils, which are optionally fluorinated, silicone oils and perfluorinated oils, alone or as a mixture.

9. Use according to any one of the preceding claims, in which the emulsion is an oil-in-water or water-in-oil emulsion or a multiple emulsion.

10. Use according to any one of the preceding claims, in which the aqueous phase/oily phase ratio is between 9 and 0.1 and preferably between 5 and 1.5.

11. Use according to any one of the preceding claims, in a or for the preparation of a cosmetic, hygiene, pharmaceutical or food product.

12. Use according to any one of the preceding claims, in a or for the preparation of a product for making up and/or for caring for the skin or keratinous substances or a product for dyeing the hair or a product for protecting the skin and/or keratinous substances from the sun.

13. Emulsion comprising a fatty phase and an aqueous phase, characterized in that the fatty phase comprises, as sole liquid oil, at least 50% by weight, with respect to the total weight of the fatty phase, of at least one fluorohydrocarbon compound of formula (I) as defined in one of Claims 1 to 4.

14. Emulsion according to Claim 13, in which the compound of formula (I), or the mixture of these compounds, represents at least 75 % by weight of the fatty phase.

15. Emulsion according to one of Claims 13 and 14, in which the fatty phase additionally comprises at least one fat-soluble additive preferably chosen from vitamins, fragrances, fatty acids, lipids or ceramides.

16. Emulsion according to one of Claims 13 to 15, in which the aqueous phase/oily phase ratio is between 9 and 0.1 and preferably between 5 and 1.5.

17. Emulsion according to one of Claims 13 to 16, which is provided in the water-in-oil or oil-in-water form or in the form of a multiple emulsion.

18. Cosmetic, hygiene, pharmaceutical or food composition, characterized in that it comprises an emulsion according to one of Claims 13 to 17.

19. Composition according to Claim 18, which is provided in the form of a product for making up and/or for caring for the skin or keratinous substances or in the form of a product for dyeing the hair or of a product for protecting the skin and/or keratinous substances from the sun.

## Patentansprüche

1. Verwendung einer oder mehrerer Fluorkohlenwasserstoffverbindungen der Formel (I):
R_{F}-C₂H₄-O-CO-R_{H} (I),
worin bedeuten:
R_{F} eine geradkettige oder verzweigte perfluorierte Alkylgruppe mit 4 bis 20 Kohlenstoffatomen und
R_{H} eine geradkettige oder verzweigte Alkylgruppe mit 1 bis 29 Kohlenstoffatomen,
als Stabilisierungsmittel in einer Emulsion, die mindestens eine Fettphase und eine wäßrige Phase enthält.

2. Verwendung nach Anspruch 1, wobei R_{F} eine perfluorierte Alkylgruppe mit 4 bis 10 Kohlenstoffatomen bedeutet.

3. Verwendung nach einem der vorhergehenden Ansprüche, wobei R_{H} eine geradkettige oder verzweigte Alkylgruppe mit 1 bis 15 Kohlenstoffatomen bedeutet.

4. Verwendung nach einem der vorhergehenden Ansprüche, wobei die Verbindung der Formel (I) ausgewählt ist unter: Hexansäure-2-perfluoroctyl-ethylester, 2-Butyl-octansäure-2'-perfluorhexyl-ethylester, 2-Ethyl-hexansäure-2'-perfluorhexyl-ethylester, 2-Decyl-tetradecansäure-2'-perfluoroctyl-ethylester, Octansäure-2-perfluorhexyl-ethylester, Octansäure-2-perfluoroctyl-ethylester, Octadecansäure-2-perfluoroctyl-ethylester, Docosansäure-2-perfluoroctyl-ethylester und Decansäure-2-perfluoroctyl-ethylester.

5. Verwendung nach einem der vorhergehenden Ansprüche, wobei das Stabilisierungsmittel in der Fettphase der Emulsion vorliegt.

6. Verwendung nach einem der vorhergehenden Ansprüche, wobei die Verbindung der Formel (I) in der Emulsion in einem Mengenanteil von 0,1 bis 30 Gew.-% vorliegt.

7. Verwendung nach Anspruch 6, wobei die Verbindung der Formel (I) in der Emulsion in einem Mengenanteil von 0,5 bis 20 Gew.-% vorliegt.

8. Verwendung nach einem der vorhergehenden Ansprüche, wobei die Fettphase der Emulsion mindestens ein Öl enthält, das unter Kohlenwasserstoffölen, die gegebenenfalls fluoriert sind, Siliconölen und perfluorierten Ölen sowie entsprechenden Gemischen ausgewählt ist.

9. Verwendung nach einem der vorhergehenden Ansprüche, wobei die Emulsion eine Öl-in-Wasser-Emulsion oder Wasser-in-Öl-Emulsion oder eine multiple Emulsion ist.

10. Verwendung nach einem der vorhergehenden Ansprüche, wobei das Verhältnis von wäßriger Phase zu Fettphase im Bereich von 9 bis 0,1 und vorzugsweise von 5 bis 1,5 liegt.

11. Verwendung nach einem der vorhergehenden Ansprüche in kosmetischen, hygienischen oder pharmazeutischen Produkten oder Lebensmitteln oder zur Herstellung von kosmetischen, hygienischen oder pharmazeutischen Produkten oder Lebensmitteln.

12. Verwendung nach einem der vorhergehenden Ansprüche in Produkten zum Schminken und/oder zur Pflege der Haut oder von Keratinfasern oder Produkten zur Färbung der Haare oder Produkten zum Sonnenschutz der Haut und/oder von Keratinfasern oder zur Herstellung derartiger Produkte.

13. Emulsion, die eine Fettphase und eine wäßrige Phase enthält, dadurch gekennzeichnet, daß die Fettphase als einziges flüssiges Öl mindestens 50 Gew.-% mindestens einer Fluorkohlenwasserstoffverbindung der Formel (I) nach einem der Ansprüche 1 bis 4, bezogen auf das Gesamtgewicht der Fettphase, enthält.

14. Emulsion nach Anspruch 13, wobei die Verbindung der Formel (I) oder das Gemisch dieser Verbindungen mindestens 75 Gew.-% der Fettphase ausmacht.

15. Emulsion nach einem der Ansprüche 13 oder 14, wobei die Fettphase ferner mindestens einen öllöslichen Hilfstoff enthält, der vorzugsweise unter den Vitaminen, Parfums, Fettsäuren, Lipiden und Ceramiden ausgewählt ist.

16. Emulsion nach einem der Ansprüche 13 bis 15, wobei das Verhältnis von wäßriger Phase zu Ölphase im Bereich von 9 bis 0,1 und vorzugsweise von 5 bis 1,5 liegt.

17. Emulsion nach einem der Ansprüche 13 bis 16, die in Form einer Wasser-in-Öl-Emulsion oder Öl-in-Wasser-Emulsion oder in Form einer multiplen Emulsion vorliegt.

18. Kosmetische, hygienische oder pharmazeutische Zusammensetzung oder Lebensmittelzusammensetzung, dadurch gekennzeichnet, daß sie aus einer Emulsion nach einem der Ansprüche 13 bis 17 besteht.

19. Zusammensetzung nach Anspruch 18, die in Form eines Produkts zum Schminken und/oder zur Pflege der Haut oder von Keratinfasern oder in Form eines Produkts zum Färben von Haaren oder eines Produkts zum Sonnenschutz der Haut und/oder von Keratinfasern vorliegt.
